# NEUE EUROPÄISCHE PATENTSCHRIFT

(11) **EP 1 395 117 B2**
(45) Veröffentlichungstag und Bekanntmachung des Hinweises auf die Entscheidung über den Einspruch: **30.09.2009**
(45) Hinweis auf die Patenterteilung: 26.07.2006
(21) Anmeldenummer: 02724307.0
(22) Anmeldetag: 06.05.2002
(51) Int. Cl.: A01N 43/90, B27K 3/34, C09D 5/14, C08K 5/3477

(54) **VERWENDUNG VON TRIAZOLOPYRIMIDIN-DERIVATEN ALS MIKROBIZIDE IM MATERIALSCHUTZ**
USE OF TRIAZOLOPYRIMIDINE DERIVATIVES AS MICROBICIDES IN THE PROTECTION OF MATERIALS
UTILISATION DE DERIVES DE TRIAZOLOPYRIMIDINE EN TANT QUE MICROBICIDES POUR LA PROTECTION DE MATERIEL

(30) Priorität: 18.05.2001 DE 10124208
(43) Veröffentlichungstag der Anmeldung: 10.03.2004
(73) Patentinhaber: LANXESS Deutschland GmbH, 51369 Leverkusen (DE)
(72) Erfinder: BRUNS, Rainer, 51373 Leverkusen (DE); KUGLER, Martin, 42799 Leichlingen (DE); JÄTSCH, Thomas, 50670 Köln (DE); ELBE, Hans-Ludwig, 42329 Wuppertal (DE); KUHNT, Dietmar, 51399 Burscheid (DE); GEBAUER, Olaf, 50737 Köln (DE); RIECK, Heiko, 69110 Ste. Foy-lès-Lyon (FR)
(86) Internationale Anmeldenummer: PCT/EP2002/004965
(87) Internationale Veröffentlichungsnummer: WO 2002/094020

(56) Entgegenhaltungen:
- EP-A- 0 336 186
- EP-A- 0 550 113
- EP-A- 0 556 671
- WO-A-02/50077
- WO-A1-19/98046607
- WO-A1-20/02083676
- WO-A2-20/02002563
- US-A- 5 985 883
- B. ELVERS, S. HAWKINS, G. SCHULZ: 'Ullmann's Encycl.of Ind. Chem.', Bd. A16, 1990, VCH VERLAGSGESELL.GMBH, WEINHEIM, DE, ISBN 3-527-20116-5 Artikel K. R. PAYNE AND E. HILL: 'Microbiocides', Seiten 563 - 564
- ED. B. ELVERS, S. HAWKINS: 'Ullmann's Encycl. of Ind. Chem.', Bd. A 28, 1990, VCH VERLAGSGES. GMBH, WEINHEIM, DE, ISBN 3-527-20128-9 Artikel E.-H. POMMER: 'Wood, Preservation', Seiten 357 - 366
- KIRK-OTHMER: 'Enc. of Chem. Technology', Bd. 6, 1993, JOHN WILEY & SONS, NEW YORK, US, ISBN 0-471-52674-6 Artikel 'Coatings', Seiten 669 - 718
- KIRK OTHMER: 'Enc. of Chem. Technology', Bd. 14, 1995, JOHN WILEY & SONS, NEW YORK, US, ISBN 0-471-52683-5 Artikel 'Industrial Antimicrobial Agents', Seiten 174 - 177

## Beschreibung

Die vorliegende Anmeldung betrifft neue Verwendung von bekannten Triazolopyrimidin-Derivaten als Mikrobizide zum Schutz von technischen Materialien.

Aus EP-A 550 113 sind bereits Triazolopyrimidin-Derivate bekannt, deren Pyrimidinring in 7-Stellung durch eine Aminogruppe -NR¹R², in 6-Stellung durch gegebenenfalls substituiertes Phenyl oder Naphthyl und in 5-Stellung durch Halogen oder einen Rest -NR⁵R⁶ substituiert ist. Die dort beschriebenen Verbindungen eignen sich zum Schutz von Pflanzen vor dem Befall von phytopathogenen Pilzen.

US-A-5 985 883 beschreibt ebenfalls Triazolopyrimidin-Derivate, die in 6-Stellung des Pyrimidinringes durch 2,4,6-Trichlorophenyl substituiert sind, zum Schutz von Pflanzen vor dem Befall durch phytopathoge Pilze.

WO 02/50077 A2, veröffentlicht nach dem Anmeldedatum der vorliegenden Anmeldung und somit nur relevant unter Art. 54(3) EPÜ, offenbart Triazolopyrimidin-Derivate sowie deren Verwendung zur Bekämpfung von unerwünschten Mikroorganismen im Pflanzen- und Materialschutz. Die eingesetzten Triazolopyrimidine unterscheiden sich aber von den erfindungsgemäß eingesetzten Verbindungen, insbesondere im Substituent R³. WO02/083676 A1, veröffentlicht nach dem Anmeldedatum der vorliegenden Anmeldung und somit nur relevant unter Art. 54(3)EPÜ, offenbart Triazolopyrimidin-Derivate mit Trifluoropropylaminogruppen zum Schutz von Holz, Papier, Dispersionsfarben, Fasern und Gewebe.

Es wurde nun überraschenderweise gefunden, dass die vorbeschriebenen Triazolopyrimidin-Derivate eine besonders gute und breite mikrobizide Wirkung gegen die zum Schutz von technischen Materialien relevanten Mikroorganismen zeigen. Dieser Befund ist insbesondere überraschend, da sich einerseits die betreffenden Organismen grundlegend von den phytopathogen Pilzen unterscheiden und andererseits der Schutz von technischen Materialien grundlegend andere Anforderungen an die Substanzen bzgl. ihrer Stabilität, ihres Leachingverhaltens, ihrer Farbigkeit, und ihrer Kompatibilität zu den prinzipiell anderen Formulierhilfsmitteln stellt.

Außerdem wurde gefunden, dass die erfindungsgemäß einzusetzenden Verbindungen eine hohe Stabilität in technischen Medien aufweisen.

Gegenstand der vorliegenden Erfindung ist die Verwendung von Triazolopyrimidin-Derivaten der Formel (I) 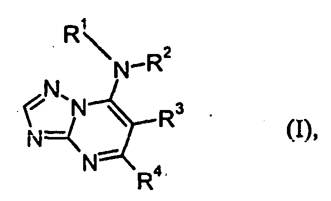 worin
- R¹: für Methyl, Ethyl, Propyl, Trifluoropropyl, 2-(1,1,1-Trifluoropropyl), Benzyl, Pentenyl, Propinyl, Cyclopropyl, Cyclopentyl, Trimethylcyclopentyl, Cyclohexyl, Trimethylcyclohexyl oder Cyclooctyl, steht,
- R²: für Wasserstoff, Methyl oder Ethyl steht,
oder
- R¹ und R²: zusammen mit dem Stickstoffatom an welches sie gebunden sind für Piperidyl, Phenylpiperidyl, Methylpiperidyl oder Azepinyl stehen,
- R³: für Phenyl, 2-Fluorophenyl, 3-Fluorophenyl, 4-Fluorophenyl, 2-Chlorophenyl, 3-Chlorophenyl, 4-Chlorophenyl, 2-Bromophenyl, 3-Bromophenyl, 4-Bromophenyl, 2-Chloro-6-Fluorophenyl, 2,4-Difluorophenyl, 3,4-Difluorophenyl, 2,6-Difluorophenyl, 2,4,6-Trifluorophenyl, 2,3,6-Trifluorophenyl, 2,4-Dichlorophenyl, 3,4-Dichlorophenyl, 2,6-Dichlorophenyl, 2,4,6-Trichlorophenyl, 2-Methylphenyl, 3-Methylphenyl, 4-Methylphenyl, 3-Butylphenyl, 4-Butylphenyl, 2-Methoxyphenyl, 3-Methoxyphenyl, 4-Methoxyphenyl, 3,4-Dimethoxyphenyl oder 2,6-Difluoro-4-methoxyphenyl steht, und
- R⁴: für Chlor steht,
deren Metallsalze, Säureadditionsverbindungen, N-Oxide, (*R*)- und (*S*)-Isomere sowie deren Racemate, sofern ein chirales Zentrum in den Verbindungen der Formel (I) vorhanden ist, als Mikrobizid
zum Schutz von technischen Materialien mit der Maßgabe, dass zum Schutz von Holz, Papier, Dispersionsfarben, Fasern und Gewebe R1 nicht für Trifluorpropyl steht.

Gegenstand der vorliegenden Erfindung sind ebenso die Verwendung der Metallsalze, der Säureadditionsverbindungen, der N-Oxide, der, sofern ein Chiralitätszentrum vorhanden ist, gegebenenfalls angereicherten (*R*)- und (*S*)-Isomere als auch deren Racemate, der Verbindungen mit der allgemeinen Formel (I) als Mikrobiozide zum Schutz von technischen Materialien.

Als Metallsalz kommen vorzugsweise Salze von Metallen der II. bis IV. Hauptgruppe und der I. und II. sowie der IV. bis VIII. Nebengruppe des Periodensystems in Frage, wobei Kupfer, Zink, Mangan, Magnesium, Zinn, Eisen, Calcium, Aluminium, Blei, Chrom, Kobalt und Nickel, beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalzkomplexe der Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann die Metallsalz-Komplexe in bekannter Weise, z.B. durch Abfiltrieren isolieren und gegebenenfalls durch Umkristallisieren reinigen.

Zur Herstellung von Säureadditionsverbindungen der Verbindungen der allgemeinen Formel (I) kommen vorzugsweise folgende Säuren in Frage: Die Halogenwasserstoffsäuren, wie z.B. Chlorwasserstoffsäure und Bromwasserstoffsäure" insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Propionsäure, 2-Ethylhexansäure, Buttersäure, Mandelsäure, Oxalsäure, Bemsteinsäure, 2-Hydroxy-ethan-dicarbonsäure, Maleinsäure, Fumarsäure, Weinsäure, Citronensäure, Salicylsäure, Sorbinsäure, Milchsäure sowie Sulfonsäuren, wie z.B. p-Toluolsulfonsäure, 1,5-Naphthalindisulfonsäure, Alkansulfonsäuren, Benzoesäure und gegebenenfalls substituierte Benzoesäuren.

Die Säureadditions-Salze der Verbindungen der allgemeinen Formel (I) können in einfacher Weise nach üblichen Salzbildungsmethoden, z.B. durch Lösung einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäß verwendbaren Stoffe der Formel (I) weisen überraschenderweise eine starke mikrobizide Wirkung auf und können zur Bekämpfung von unerwünschten Mikroorganismen, wie Fungi und Bakterien, im Materialschutz eingesetzt werden.

Im Materialschutz lassen sich die erfindungsgemäßen Stoffe zum Schutz von technischen Materialien gegen Befall und Zerstörung durch unerwünschte Mikroorganismen einsetzen. Unter technischen Materialien sind im vorliegenden Zusammenhang nichtlebende Materialien zu verstehen, die für die Verwendung in der Technik zubereitet worden sind. Beispielsweise können technische Materialien, die durch diese Erfindung vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Die Wirkstoffe der Formel (I) sowie diese enthaltene Mittel bzw. Konzentrate sowie Mischungen werden vorzugsweise zum Schutz von Holz und Holzwerkstoffen gegen Mikroorganismen, z.B. gegen holzzerstörende oder holzverfärbende Organismen, insbesondere Pilze verwendet.

Unter Holz, welches durch die Verbindungen der Formel (I) bzw. diese enthaltene Mischungen geschützt werden kann, ist beispielsweise, jedoch nicht'ausschließlich, zu verstehen: Bauholz, Holzbalken, Eisenbahnschwellen, Brückenteile, Bootsstege, Holzfahrzeuge, Kisten, Paletten, Container, Telefonmasten, Holzzäune, Holzverkleidungen, Holzfemster und -türen, Sperrholz, Spanplatten, Tischlerarbeiten und Holzprodukte, die beim Hausbau oder in der Bautischlerei Verwendung finden.

Ein besonders effektiver Holzschutz wird durch großtechnische Imprägnierverfahren, z.B. Vakuum-, Doppelvakuum- oder Druckverfahren, erzielt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze sowie holzverfärbende und holzzerstörende Pilze.

Es seien beispielsweise Mikroorganismen der folgenden Gattungen genannt:
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe der Formel (I) können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen sowie ULV-Kalt- und Warmnebel-Formulierungen.

Die Formulierungen bzw. Mittel zum Schutz von technischen Materialien werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycerol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter normalem Druck gasförmig sind, z.B.

Aerosol-Treibgase, wie Halogenkohlenwasserstoffe sowie Butan, Propan, Stickstoff und Kohlendioxid. Als feste Träger kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bimns, Sepiolit, Dolomit sowie synthetische Granulate aus anorganischem und organischem Mehlen sowie Granulate aus organischem Material wie Sägemehl, Kokosnussschalen, Maiskolben und Tabakstengeln. Als Emulgier- und/oder schaumerzeugende Mittel kommen in Frage: z.B. nichtionogene und anionogene Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Ligninsulfitablaugungen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalcycaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0.1 und 95 Gewichtsprozent Wirkstoff, vorzugsweise zwischen 0.5 und 90%.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Hierbei erweisen sich die folgenden Mischpartner als besonders günstig:

### Triazole wie:

Azaconazole, Azocyclotin, Bitertanol, Bromuconazole, Cyproconazole, Diclobutrazole, Difenoconazole, Diniconazole, Epoxyconazole, Etaconazole, Fenbuconazole, Fenchlorazole, Fenethanil, Fluquinconazole, Flusilazole, flutriafol; Furconazole, Hexaconazole, Imibenconazle, Ipconazole, Isozofos, Myclobutanil, Metconazole, Paclobutrazol, Penconazole, Propioconazole, (±)-cis-1-(4-chlorphenyl)-2-(1H-1,2,4-triazol-1-yl)-cycloheptanol, 2-(1-tert.-Butyl)-1-(2-chlorphenyl)-3-(1,2,4-triazol-1-yl)-propan-2-ol, Tebuconazole, Tetraconazole, Triadimefon, Triadimenol, Triapenthenol, Triflumizole, Triticonazole, Uniconazole sowie deren Metallsalze und Säureaddukte;

### Imidazole wie:

Clotrimazole, Bifonazole, Climbazole, Econazole, Fenapamil, Imazalil, Isoconazole, Ketoconazole, Lombazole, Miconazole, Pefurazoate, Prochloraz, Triflumizole, Thiazolcar, 1-Imidazolyl-1-(4'-chlorophenoxy)-3,3-dimethylbutan-2-on sowie deren Metallsalze und Säureaddukte;

### Pyridine und Pyrimidine wie:

Ancymidol, Buthiobate, Fenarimol, Mepanipyrin, Nuarimol, Pyroxyfur, Triamirol;

### Succinat-Dehydrogenase Inhibitoren wie:

Benodanil, Carboxim, Carboximsulfoxid, Cyclaflluramid, Fenfuram, Flutanil, Furcabanil, Furmecyclox, Mebenil, Mepronil, Methfuroxam, Metsulfovax, Pyrocarbolid, Oxycarboxin, Shirlan, Seedvax;

### Naphthalin-Derivate wie:

Terbinafine, Naftifine, Butenafine, 3-Chloro-7(2-aza-2,7,7-trimethyl-oct-3-en-5-in);

### Sulfenamide wie:

Dichlofluanid, Tolylfluanid, Folpet, Fluorfolpet; Captan, Captofol;

### Benzimidazole wie:

Carbendazim, Benomyl, Fuberidazole, Thiabendazole oder deren Salze;

### Morpholinderivate wie:

Aldimorph, Dimethomorph, Dodemorph, Falimorph, Fenpropidin, Fenpropimorph, Tridemorph, Trimorphamid und ihre Arylsulfonsäuren Salze, wie z.B. p-Toluolsulfonsäure und p-Dodecylphenyl-sulfonsäure;

### Benzthiazole wie:

2-Mercaptobenzothiazol;

### Benzthiophendioxide wie:

Benzo[b]thiophen-S,S-dioxid-carbonsäurecyclohexylamid;

### Benzamide wie:

2,6-Dichloro-N-(4-trifluoromethylbenzyl)-benzamide, Tecloftalam;

### Borverbindungen wie:

Borsäure, Borsäureester, Borax;

### Formaldehyd und Formaldehydabspaltende Verbindungen wie:

Benzylalkoholmono-(poly)-hemiformal, n-Butanol-hemiformal, Dazomet, Ethylenglycolhemiformal, hexa-hydro-S-triazine, Hexamethylentetramin, N-Hydroxymethyl-N'-methylthioharnstoff, N-Methylolchloracetamid, Oxazolidine, Paraformaldehyd, Taurolin, Tetrahydro-1,3-oxazin, N-(2-Hydroxypropyl)-amin-methanol;

### Isothiazolinone wie:

N-Methylisothiazolin-3-on, 5-Chlor-N-methylisothiazolin-3-on, 4,5-Dichloro-N-octylisothiazolin-3-on, 5-Chlor-N-octylisothiazolinen, N-Octyl-isothiazolin-3-on, 4,5-Trimethylen-isothiazolinone, 4,5-Benzisothiazolinone;

### Aldehyde wie:

Zimtaldehyd, Formaldehyd, Glutardialdehyd, β-Bromzimtaldehyd;

### Thiocyanate wie:

Thiocyanatomethylthiobenzothiazol, Methylenbisthiocyanat;

### quartäre Ammoniumverbindungen und Guanidine wie:

Benzalkoniumchlorid, Benzyldimethyltetradecylammoniumchlorid, Benzyldimethyldodecyl-ammoniumchlorid, Dichlorbenzyl-dimethyl-alkyl-ammoniumchlorid, Didecyldimethylammmoniumchlorid, Dioctyl-dimethyl-ammoniumchlorid, N-Hexadecyltrimethyl-ammoniumchlorid, 1-Hexadecyl-pyridinium-chlorid, Iminoctadine-tris(albesilate);

### Iodderivate wie:

Diiodmethyl-p-tolylsulfon, 3-Iod-2-propinyl-alkohol, 4-Chlorphenyl-3-iodpropargylformat, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyclohexylcarbamat, 3-Iod-2-propinylphenylcarbamat;

### Phenole wie:

Tribromphenol, Tetrachlorphenol, 3-Methyl-4-chlorphenol, 3,5-Dimethyl-4-chlorphenol, Phenoxyethanol, Dichlorphen, 2- Benzyl-4-chlorphenol, 5-Chlor-2-(2,4-dichlorphenoxy)-phenol, Hexachlorophen, p-Hydroxybenzoesäureester, o-Phenylphenol, m-Phenylphenol, p-Phenylphenol und deren Alkali- und Erdalkalimetallsalze;

### Mikrobizide mit aktivierter Halogengruppe wie:

Bronidox, 2-Brom-2-nitro-1,3-propandiol, 2-Brom-4'-hydroxy-acetophenon, 1-Brom-3-chlor-4,4,5,5-tetramethyl-2-imidazoldinone, β-Brom-β-nitrostyrol, Chloracetamid, Chloramin T, 1,3-Dibrom-4,4,5,5-tetramethyl-2-imidazoldinone, Dichloramin T, 3,4-Dichlor-(3H)-1,2-dithiol-3-on, 2,2-Dibrom-3-nitril-propionamid, 1,2-Dibrom-2,4-dicyanobutan, Halane, Halazone, Mucochlorsäure, Phenyl-(2-chlor-cyan-vinyl)sulfon, Phenyl-(1,2,dichlor-2-cyanvinyl)sulfon, Trichlorisocyanursäure;

### Pyridine wie:

1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2( 1H)-pyridin;

### Methoxyacrylate oder ähnliches wie:

Azoxystrobin,
Methyl-(E)-methoximino[alpha-(o-tolyloxy)-o-tolyl]acetat,
(E)-2-Methoxyimino-N-methyl-2-(2-phenoxyphenyl)acetamid,
(E)-2- {2-[6-(2-cyanophenoxy)pyrimidin-4-yloxy]phenyl}-3-methoxyacrylat,
O-Methyl-2-[[[[[[3-methoximino-2-butyl]imino]imino]oxy]o-tolyl]-2-methoximinoacetimidate,
2-[[[[1-(2,5-dimethylphenyl)ethylidene]amino]oxy]methyl]-alpha-(methoximino)-N-methylbenzeneacetamide,
alpha-(methoxyimino)-N-methyl-2-[[[[1-[3-(trifluoromethyl)phenyl]-ethylidene]-amino]oxy]methyl]-benzeneacetamide,
Trifluoxystrobin,
alpha-(methoxymethylene)-2-[[[[1-[trifluoromethyl)phenyl]ethylidene]amino]-oxy]methyl]benzeneaceticacid-methylester,
2-[[[5-chloro-3-(trifluormethyl)-2-pyridinyl]oxy]methyl]-alpha-(methoxyimino)-N-methylbenzeneacetamide,
2-[[[cyclopropyl[(4-ethoxyphenyl)imino]methyl]thio]methyl]-alpha-(methoxyimino)-benzeneaceticacid-methylester,
alpha-(methoxyimino)-N-methyl-2-)4-methyl-5-phenyl-2,7-dioxa-3,6-diazaocta-3,5-dien-1-yl)-benzeneacetamide,
alpha-(methoxymethylene)-2-(4-methyl-5-phenyl-2,7-dioxa-3,6-diazaocta-3,5-dien-1-yl-benzeneaceticacid-methylester,
alpha(-methoximino)-N-methyl-2-[[[1-[3-trifluoromethyl)phenyl]ethoxy]imino]-methyl]-benzeneacetamide,e
2-[[(3,5-dichloro-2-pyridinyl)-oxy]methyl]-alpha-(methoxyimino)-N-methylbenzeneacetamide,
2-[4,5-dimethyl-9-(4-morpholinyl)-2,7-dioxa-3,6-diazanona-3,5-dien-1-yl]-alpha-(methoxymethylene)-benzeneaceticacid-methylester,
Kresoxim-methyl;

### Metallseifen wie:

Zinn-, Kupfer-, Zinknaphtenat, -octoat, 2-ethylhexanoat, -oleat, -phosphat, -benzoat;

### Metallsalze wie:

Kupferhydroxycarbonat, Natriumdichromat, Kaliumdichromat, Kaliumchromat, Kupfersulfat, Kupferchlorid, Kuperborat, Zinkfluorosilikat, Kupferfluorosilikat;

### Oxide wie:

Tributylzinnoxid, Cu₂O, CuO, ZnO;

### Dithiocarbamate wie:

Cufraneb, Ferban, Kalium-N-hydroxymethyl-N'-methyl-dithiobarbamat, Na- oder K-dimethyldithiocarbamat, Macozeb, Maneb, Metam, Metiram, Thiram, Zineb, Ziram;

### Nitrile wie:

2,4,5,6-Tetrachlorisophthalodinitril, Dinatrium-cyano-dithioimidocarbamat;

### Chinoline wie:

8-Hydroxychinolin und deren Cu-Salze;

### sonstige Fungizide und Bakterizide wie:

5-Hydroxy-2(5H)-furanon; 4,5-Benzdithiazolinon, 4,5-Trimethylendithiazolinon, N-(2-p-Chlorbenzoylethyl)-hexaminiumchlorid, 2-Oxo-2-(4-hydroxy-phenyl)acethydroximsäurechlorid, Tris-N-(cyclohexyldiazeniumdioxy)-aluminium, N-(Cyclohexyldiazeniumdioxy)-tri-butylzinn bzw. K-Salze, Bis-N-(cyclohexyldiazeniumdioxy)-kupfer, Iprovalicarb, Fenhexamid, Spiroxamine, Carpropamid, Diflumetorin, Quinoxyfen, Famoxadone, Polyoxorim, Acibenzolar-S-methyl, Furametpyr, Thifluzamide, Methalaxy-M, Ag, Zn oder Cu-haltige Zeolithe allein oder eingeschlossen in polymere Werkstoffe.

Ganz besonders bevorzugt sind Mischungen mit
Azaconazole, Bromuconazole, Cyproconazole, Dichlobutrazol, Diniconazole, Hexaconazole, Metaconazole, Penconazole, Propiconazole, Tebuconazole, Dichlofluanid, Tolylfluanid, Fluorfolpet, Methfuroxam, Carboxin, Benzo[b]thiophen-S,S-dioxidcarbonsäurecyclohexylamid, Fenpiclonil, 4-(2,2-Difluoro-1,3-benzodioxol-4-yl)-1H-pyrrol-3-carbonitril, Butenafine, Imazalil, N-Methyl-isothiazolin-3-on, 5-Chlor-N-methylisothiazolin-3-on, N-Octylisothiazolin-3-on, Dichlor-N-octylisothiazolinon, Mercaptobenthiazol, Thiocyanatomethylthiobenzothiazol, Benzisothiazolinone, N-(2-Hydroxypropyl)-amino-methanol, Benzylalkohol-(hemi)-formal, N-methylolchloracetamid, N-(2-Hydroxypropyl)-amin-methanol, Glutaraldehyd, Omadine, Dimethyldicarbonat, 2-Brom-2-nitro-1,3-propandiol und/oder 3-Iodo-2-propinyl-n-butylcarbamate.

Des weiteren werden neben den oben genannten Fungiziden und Bakteriziden auch gut wirksame Mischungen mit anderen Wirkstoffen hergestellt:

### Insektizide / Akarizide / Nematizide:

Abamectin, Acephate, Acetamiprid, Acrinathrin, Alanycarb, Aldicarb, Aldoxycarb, Aldrin, Allethrin, Alpha-cypermethrin, Amitraz, Avermectin, AZ 60541, Azadirachtin, Azinphos A, Azinphos M, Azocyclotin,
Bacillus thuringiensis, Barthrin, 4-Bromo-2(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, Bioresmethrin, Bioallethrin, Bromophos A, Bromophos M, Bufencarb, Buprofezin, Butathiophos, Butocarboxim, Butoxycarboxim,
Cadusafos, Carbaryl, Carbofuran, Carbophenothion, Carbosulfan, Cartap, Chinomethionat, Chloethocarb, Chlordane, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpicrin, Chlorpyrifos A, Chlorpyrifos M, Cis-Resmethrin, Clocythrin, Cypophenothrin, Clofentezin, Coumaphos, Cyanophos, Cycloprothrin, Cyfluthrin, Cyhalothrin, Cyhexatin, Cypermethrin, Cyromazin,
Decamethrin, Deltamethrin, Demeton M, Demeton S, Demeton-S-methyl, Diafenthiuron, Dialiphos, Diazinon, 1,2-Dibenzoyl-1(1,1-dimethyl)-hydrazin, DNOC, Dichlofenthion, Dichlorvos, Dicliphos, Dicrotophos, Difethialone, Diflubenzuron, Dimethoat, Dimethyl-(phenyl)-silyl-methyl-3-phenoxybenzylether, Dimethyl-(4-Ethoxyphenyl)-silylmethyl-3-phenoxybenzylether, Dimethylvinphos, Dioxathion, Disulfoton,
Eflusilanate, Emamectin, Empenthrin, Endosulfan, EPN, Esfenvalerat, Ethiofencarb, Ethion, Ethofenprox, Etrimphos, Etoxazole, Etobenzanid,
Fenamiphos, Fenazaquin, Fenbutatinoxid, Fenfluthrin, Fenitrothion, Fenobucarb, Fenothiocarb, Fenoxycarb, Fenpropathrin, Fenpyrad, Fenpyroximat, Fensulfothion, Fenthion, Fenvalerate, Fipronil, Fluazuron, Flucycloxuron, Flucythrinate, Flufenoxuron, Flupyrazofos, Flufenzine, Flumenthrin, Flufenprox, Fluvalinate, Fonophos, Formethanate, Formothion, Fosmethilan, Fosthiazat, Fubfenprox, Furathiocarb, Halofenocid, HCH, heptenophos, Hexaflumuron, Hexythiazox, Hydramethylnon, Hydroprene,
Imidacloprid, Imiprothrin, Indoxycarb, Iodfenfos, Iprinomectin, Iprobenfos, Isaophos, Isoamidophos, Isofenphos, Isoprocarb, Isoprothiolane, Isoxathion, Ivermectin, Lamacyhalothrin, Lufenuron,
Kadedrin,
Lambda-Cyhalothrin, Lufenuron,
Malathion, Mecarbam, Mervinphos, Mesulfenphos, Metaldehyd, Methacrifos, Methamidophos, Methidathion, Methiocarb, Methomyl, Metalcarb, Milbemectin, Monocrotophos, Moxiectin,
Naled, NC 184, NI 125, Nicotin, Nitenpyram,
Omethoat, Oxamyl, Oxydemethon M, Oxydeprofos,
Parathion A, Parathion M, Penfluron, Permethrin, 2-4-Phenoxyphenoxy)ethylethylcarbamat, Phenthoat, Phorat, Phosalon, Phosmet, Phosphamidon, Phoxim, Pirimicarb, Pirimiphos M, Pirimiphos A, Prallethrin, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen, Pyrithiobac-natrium,
Quinalphos,
Resmethrin, RH-7988, Rotenone,
Salithion, Sebufos, Silafluofen, Spinosad, Sulfotep, Sulprofos,
Tau-fluvalinate, Taroils, Tebufenozide, Tebufenpyrad, Tebupirimphos, Teflubenzuron, Tefluthrin, Temephos, Terbam, Terbufos, Tetrachlorvinphos, Tetramethrin, Tetramethacarb, Thiacloprid, Thiafenox, Thiamethoxam, Thiapronil, thiodicarb, Thiofanox, Thiazophos, Thiocyclam, Thiomethon, Thionazin, Thuringiensin, Tralomethrin, Transfluthrin, Triarathen, Triazophos, Triazamate, Trichlorfon, Triflumuron, Trimethacarb,
Vamidothion, XMC, Xylylcarb, Zetamethrin;

### Molluscizide:

Fentinacetate, Metaldehyde, Methiocarb, Niclosamide;

### Herbizide und Algizide:

Acetochlor, Acifluorfen, Aclonifen, Acrolein, alachlor, Alloxydim, Ametryn, Amidosulfuron, Amitrole, Ammonium sulfamate, Anilofos, Asulam, Atrazine, Azafenidin, Aziptrotryne, Azimsulfuron,
Benazolin, Benfluralin, Benfuresate, Bensulfuron, Bensulfide, Bentazone, Benzofencap, Benzthiazuron, Bifenox, Bispyribac, Bispyribac-natrium, Bispyribac-methyl, Borax, Bromacil, Bromobutide, Bromofenoxim, Bromoxynil, Butachlor, Butamifos, Butralin, Butylate, Bialaphos, Benzoyl-prop, Bromobutide, Butroxydim,
Carbetamide, Carfentrazone-ethyl, Carfenstrole, Chlomethoxyfen, Chloramben, Chlorbromuron, Chlorflurenol, Chloridazon, Chlorimuron, Chlornitrofen, Chloroacetid acid, Chloransulam-methyl, Cinidon-ethyl, Chlorotoluron, Chloroxuron, Chlorpropham, Chlorsulfuron, Chlorthal, Chlorthiamid, Cinmethylin, Cinofulsuron, Clefoxydim, Clethodim, Clomazone, Chlomeprop, Clopyralid, Cyanamide, Cyanazine, Cycloate, Cycloxydim, Chloroxynil, Clodinafop-propargyl, Cumyluron, Clometoxyfen, Cyhalofop, Cyhalofop-butyl, Clopyrasuluron, Cyclosulfamuron, Diclosulam, Dichlorprop, Dichlorprop-P, Diclofop, Diethatyl, Difenoxuron, Difenzoquat, Diflufenican, Diflufenzopyr, Dimefuron, Dimepiperate, Dimethachlor, Dimethipin, Dinitramine, Dinoseb, Dinoseb Acetate, Dinoterb, Diphenamid, Dipropetryn, Diquat, Dithiopyr, Diduron, DNOC, DSMA, 2,4-D, Daimuron, Dalapon, Dazomet, 2,4-DB, Desmedipham, Desmetryn, Dicamba, Dichlobenil, Dimethamid, Dithiopyr, Dimethametryn, Eglinazine, Endothal, EPTC, Esprocarb, Ethalfluralin, Ethidimuron, Ethofumesate, Ethobenzanid, Ethoxyfen, Ethametsulfuron, Ethoxysulfuron, Fenoxaprop, Fenoxaprop-P, Fenuron, Flamprop, Flamprop-M, Flazasulfuron, Fluazifop, Fluazifop-P, Fuenachlor, Fluchloralin, flufenacet, Flumeturon, Fluorocglycofen, Fluoronitrofen, Flupropanate, Flurenol, Fluridone, Flurochloridone, Fluroxypyr, Fomesafen, Fosamine, Fosametine, Flamprop-isopropyl, Flamprop-isopropyl-L, Flumiclorac-pentyl, Flumipropyn, Flumioxzim, Fluratome, Flumioxzim, Flupyrsulfuron-methyl, Fluthiacet-methyl, Glyphosate, Glufosinate-ammonium,
Haloxyfop, Hexazinone,
Imazamethabenz, Isoproturon, Isoxaben, Isoxapyrifop, Imazapyr, Imazaquin, Imazethapyr, Ioxynil, Isopropalin, Imazosulfuron, Imazomox, Isoxaflutole, Imazapic, Lactofen, Lenacil, Linuron,
MCPA, MCPA-thioethyl, MCPB, Mecoprop, Mecoprop-P, Mefenacet, Mefluidide, Metam, Metamitron, Metazachlor, Methabenzthiazuron, Methazole, Methoropytryne, Methyldymron, Methylisothiocyanate, Metobromuron, Metoxuron, Metribuzin, Metsulfuron, Molinate, Manolide, Monolinuron, MSMA, Metolachlor, Metosulam, Metobenzuron, Naproanilide, Napropamide, Naptalam, Neburon, Nicosulfuron, Norflurazon, Natriumchlorat, Oxadiazon, Oxyfluorfen, Oxysulfuron, Orbencarb, Oryzalin, Oxadiargyl, Propyzamide, Prosulfocarb, Pyrazolate, Pyrazolsulfuron, Pyrazoxyfen, Pyribenzoxim, Pyributicarb, Pyridate, Paraquat, Pebulate, Pendimethalin, Pentachlorophenol, Pentoxazone, Pentanochlor, Petroleum oils, Phenmedipham, Picloram, Piperophos, Pretilachlor, Primisulforon, Prodiamine, Prometryn, Propachlor, Propanil, Propaquizafob, Propazine, Propham, Propisochlor, Pyriminobac-methyl, Pelargonsäure, Pyrithiobac, Pyraflufen-ethyl, Quinmerac, Quinocloamine, Quizalofop, Quizalofop-P, Quinchlorac,
Rimsulfuron,
Sethoxydim, Sifuron, Simazine, Simetryn, Sulfosulfuron, Sulfometuron, Sulfentrazone, Sulcotrione, Sulfosate,
Teeröle, TCA, tebutam, Tebuthiuron, Terbacil, Terbumeton, Terbuthylazine, Terbutryn, Thiazafluoron, Thifensulfuron, Thiobencarb, Thiocarbazil, Tralkoxydim, Triallate, Triasulfuron, Tribenzuron, Triclopyr, Tridiphane, Trietazine, Trifluoralin, Tycor, Thdiazimin, Thiazopyr, Triflusulfuron, Vernolate.

Die Wirkstoffe können als solche, in Form von Konzentraten oder in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden.

Die zum Schutz technischer Materialien verwendeten Mittel enthalten Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im Allgemeinen liegen die Anwendungskonzentrationen im Bereich von 0,001 bis 5 Gewichts%, vorzugsweise von 0,05 bis 1,0 Gewichts%, bezogen auf das zu schützende Material.

### Anwendungsbeispiel

### Hemmtest an Riesenkolonien von Basidiomyceten

Aus Kolonien von Gloeophyllum trabeum (P1), Coniophora puteana (P2), Poria placenta (P3), Lentinus tigrinus (P4), Coriolus versicolor (P5) und Sterum sanguinolentum (P6) wurden Mycelstücke ausgestochen und auf einem Agamährboden bei 26°C inkubiert. Die Hemmung des Hyphenwachstums auf wirkstoffhaltigen Nährboden (Wirkstoffkonzentration 6 ppm) wurden mit dem Längenwachstum ohne Wirkstoffzusatz verglichen und als prozentualle Hemmung bonitiert.

| Nr | R1 | R2 | R3 | R4 | P1 | P2 | P3 | P4 | P5 | P6 |
|---|---|---|---|---|---|---|---|---|---|---|
| 1 | Cyclopentyl | H | 3-Fluorophenyl | Cl | 80 | 100 | | | 91 | |
| 2 | Cyclopentyl | H | 2-Chlorophenyl | Cl | 100 | 100 | | 94 | 100 | 100 |
| 3 | Cyclopentyl | H | 2,6-Difluorophenyl | Cl | 100 | 100 | | 100 | 100 | 100 |
| 4 | Cyclopentyl | H | 2,4,6-Trifluorophenyl | Cl | | | | | 100 | |
| 5 | Cyclopentyl | H | Phenyl | Cl | | | | | 100 | |
| 6 | Isopropyl | H | 2,4-Dichlorophenyl | Cl | 95 | 100 | | | 95 | 100 |
| 7 | -(CH₂)₆- | | 2-Fluorophenyl | Cl | 100 | 100 | | 94 | 100 | 100 |
| 8 | -(CH₂)₆- | | 2,6-Difluorophenyl | Cl | 100 | 100 | 100 | 100 | 100 | 100 |
| 9 | -(CH₂)₂CHCH₃(CH₂)₂- | | 2,4,6-Trifluorophenyl | Cl | 100 | 100 | 100 | 100 | 100 | 100 |
| 10 | -(CH₂)₂CHCH₃(CH₂)₂- | | 2-Chloro-6-fluorophenyl | Cl | 100 | 100 | 100 | 100 | 100 | 100 |
| 11 | (*S*)-2-(1,1,1- Trifluoro)-propyl | H | 2,4,6-Trifluorophenyl | Cl | 100 | 100 | 100 | 100 | 100 | 100 |

## Patentansprüche

1. Verwendung von Verbindungen der Formel (I) 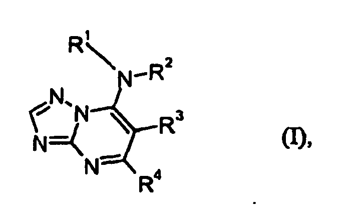 worin
R¹ für Methyl, Ethyl, Propyl, Trifluoropropyl, 2-(1,1,1-Trifluoropropyl), Benzyl, Pentenyl, Propinyl, Cyclopropyl, Cyclopentyl, Trimethylcyclopentyl, Cyclohexyl, Trimethylcyclohexyl oder Cyclooctyl, steht,
R² für Wasserstoff, Methyl oder Ethyl steht,
oder
R¹ und R² zusammen mit dem Stickstoffatom an welches sie gebunden sind für Piperidyl, Phenylpiperidyl, Methylpiperidyl oder Azepinyl stehen,
R³ für Phenyl, 2-Fluorophenyl, 3-Fluorophenyl, 4-Fluorophenyl, 2-Chlorophenyl, 3-Chlorophenyl, 4-Chlorophenyl, 2-Bromophenyl, 3-Bromophenyl, 4-Bromophenyl, 2-Chloro-6-Fluorophenyl, 2,4-Di-fluorophenyl, 3,4-Difluorophenyl, 2,6-Difluorophenyl, 2,4,6-Trifluorophenyl, 2,3,6-Trifluorophenyl, 2,4-Dichlorophenyl, 3,4-Dichlorophenyl, 2,6-Dichlorophenyl, 2,4,6-Trichlorophenyl, 2-Methylphenyl, 3-Methylphenyl, 4-Methylphenyl, 3-Butylphenyl, 4-Butylphenyl, 2-Methoxyphenyl, 3-Methoxyphenyl, 4-Methoxyphenyl, 3,4-Dimethoxyphenyl oder 2,6-Difluoro-4-methoxyphenyl steht, und
R⁴ für Chlor steht,
deren Metallsalze, Säureadditionsverbindungen, N-Oxide, (R)- und (*S*)-Isomere sowie deren Racemate, sofern ein chirales Zentrum in den Verbindungen der Formel (I) vorhanden ist,
als Mikrobizid zum Schutz von technischen Materialien, mit der Maßgabe, dass zum Schutz von Holz, Papier, Dispersionsfarben, Fasern und Gewebe R1 nicht für Trifluorpropyl steht.

2. Verwendung gemäß Anspruch 1, **dadurch gekennzeichnet, dass** als technische Materialien Holz oder Holzwerkstoffe vor dem Befall durch holzzerstörende Pilze geschützt werden.

3. Verwendung gemäß Anspruch 1, **dadurch gekennzeichnet, dass** als technische Materialien Anstrichmittel, wie z.B. Holzschutzlasuren oder Anstrichfilme vor dem Befall durch verfärbende oder Anstrichoberflächen zerstörende Pilze geschützt werden.

4. Verwendung gemäß Anspruch 1, **dadurch gekennzeichnet, dass** als technische Materialien Kunststoffe geschützt werden.

5. Verfahren zum Schutz von technischen Materialien vor Befall und / oder Zerstörung durch Mikroorganismen, **dadurch gekennzeichnet, dass** man mindestens eine Verbindung der Formel (I) gemäß Anspruch 1 auf den Mikroorganismus oder dessen Lebensraum einwirken läßt mit der Maßgabe, dass zum Schutz von Holz, Papier, Dispersionsfarben, Fasern und Gewebe R1 nicht für Trifluorpropyl steht.

6. Technische Materialien enthaltend mindestens eine Verbindung (I) gemäß Anspruch 1, mit der Maßgabe, dass zum Schutz von Holz, Papier, Dispersionsfarben, Fasern und Gewebe R1 nicht für Trifluorpropyl steht.

## Claims

1. Use of compounds of the formula (I) 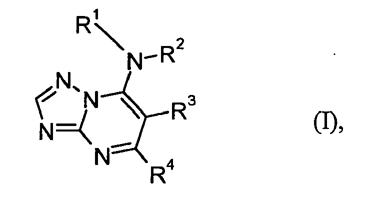 in which
R¹ represents methyl, ethyl, propyl, trifluoropropyl, 2-(1,1,1-trifluoropropyl), benzyl, pentenyl, propinyl, cyclopropyl, cyclopentyl, trimethylcyclopentyl, cyclohexyl, trimethylcyclohexyl or cyclooctyl,
R² represents hydrogen, methyl or ethyl,
or
R¹ and R² together with the nitrogen atom to which they are attached represent piperidyl, phenylpiperidyl, methylpiperidyl or azepinyl,
R³ represents phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-chloro-6-fluorophenyl, 2,4-difluorophenyl, 3,4-difluorophenyl, 2,6-difluorophenyl, 2,4,6-trifluorophenyl, 2,3,6-trifluorophenyl, 2,4-dichlorophenyl, 3,4-dichlorophenyl, 2,6-dichlorophenyl, 2,4,6-trichlorophenyl, 2-methylphenyl, 3-methylphenyl, 4-methylphenyl, 3-butylphenyl, 4-butylphenyl, 2-methoxyphenyl, 3-methoxyphenyl, 4-methoxyphenyl, 3,4-dimethoxyphenyl or 2,6-difluoro-4-methoxyphenyl, and
R⁴ represents chlorine,
their metal salts, acid addition compounds, N-oxides, (*R*)- and (*S*) isomers and, if compounds of the formula (I) contain a chiral centre, their racemates,
as microbicide for protecting engineered materials, with the proviso that in the protection of wood, paper, paint dispersions, fibres and tissues R¹ does not represent trifluoropropyl.

2. Use according to Claim 1, **characterized in that**, as engineered materials, wood or timber are protected against attack by wood-destroying fungi.

3. Use according to Claim 1, **characterized in that,** as engineered materials, paints, such as, for example, wood preservative varnishes or coating films, are protected against attack by discolouring fungi or fungi which destroy the surface of coatings.

4. Use according to Claim 1, **characterized in that**, as engineered materials, plastics are protected.

5. Process for protecting engineered materials against attack and/or destruction by microorganisms, **characterized in that** at least one compound of the formula (I) according to Claim 1 is allowed to act on the microorganism or its habitat, with the proviso that in the protection of wood, paper, paint dispersions, fibres and tissues R¹ does not represent trifluoropropyl.

6. Engineered materials, which comprise at least one compound (I) according to Claim 1, with the proviso that in the protection of wood, paper, paint dispersions, fibres and tissues R¹ does not represent trifluoropropyl.

## Revendications

1. Utilisation de composés de formule (I) 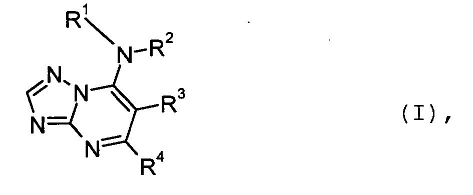 dans laquelle
R¹ représente un méthyle, un éthyle, un propyle, un trifluoropropyle, un 2-(1,1,1-trifluoropropyle), un benzyle, un pentényle, un propynyle, un cyclopropyle, un cyclopentyle, un triméthylcyclopentyle, un cyclohexyle, un triméthylcyclohexyle ou un cyclo-octyle,
R² représente un hydrogène, un méthyle ou un éthyle,
ou
R¹ et R² représentent, conjointement l'atome d'azote auquel ils sont liés, un pipéridyle, un phénylpipéridyle, un méthylpipéridyle ou un azépinyle,
R³ représente un phényle, un 2-fluorophényle, un 3-fluorophényle, un 4-fluorophényle, un 2-chlorophényle, un 3-chlorophényle, un 4-chlorophényle, un 2-bromophényle, un 3-bromophényle, un 4-bromophényle, un 2-chloro-6-fluorophényle, un 2,4-difluorophényle, un 3,4-difluorophényle, un 2,6-difluorophényle, un 2,4,6-trifluorophényle, un 2,3,6-trifluorophényle, un 2,4-dichlorophényle, un 3,4-dichlorophényle, un 2,6-dichlorophényle, un 2,4,6-trichlorophényle, un 2-méthylphényle, un 3-méthylphényle, un 4-méthylphényle, un 3-butylphényle, un 4-butylphényle, un 2-méthoxyphényle, un 3-méthoxyphényle, un 4-méthoxyphényle, un 3,4-diméthoxyphényle ou un 2,6-difluoro-4-méthoxyphényle, et
R⁴ représente un chlore,
de leurs sels métalliques, de leurs composés d'addition à un acide, de leurs N-oxydes, de leurs isomères (*R*) et (*S*) ainsi que de leurs racémates, dans la mesure où un centre chiral est présent dans les composés de formule (I),
en tant que microbicides pour la protection de matériaux industriels, à condition que pour la protection du bois, du papier, de peintures en dispersions, de fibres et de tissus, R1 ne représente pas du trifluoropropyle.

2. Utilisation selon la revendication 1, **caractérisée en ce que,** en tant que matériaux industriels, du bois ou des matériaux dérivés du bois sont protégés contre l'attaque par des champignons détruisant le bois.

3. Utilisation selon la revendication 1, **caractérisée en ce que**, en tant que matériaux industriels, des peintures, telles que, par exemple, des vernis protecteurs du bois ou des films d'enduit, sont protégés contre l'attaque par des champignons qui modifient les couleurs ou attaquent la surface d'enduits.

4. Utilisation selon la revendication 1, **caractérisée en ce que**, en tant que matériaux industriels, des matières plastiques sont protégées.

5. Procédé de protection de matériaux industriels contre l'attaque et/ou la destruction par des micro-organismes, **caractérisé en ce que** l'on laisse au moins un composé de formule (I) selon la revendication 1 agir sur le micro-organisme ou son habitat, à condition que pour la protection du bois, du papier, de peintures en dispersions, de fibres et de tissus, R1 ne représente pas du trifluoropropyle.

6. Matériaux industriels comprenant au moins un composé (I) selon la revendication 1, à condition que pour la protection du bois, du papier, de peintures en dispersions, de fibres et de tissus, R1 ne représente pas du trifluoropropyle.
